# EUROPEAN PATENT SPECIFICATION

(11) **EP 3 878 462 B1**
(45) Date of publication and mention of the grant of the patent: **26.02.2025**
(21) Application number: 21168707.4
(22) Date of filing: 29.10.2015
(51) Int. Cl.: A61K 31/513, A61K 31/675, A61K 31/704, A61K 31/7048, A61K 31/7068, A61K 38/46, A61K 45/06, A61P 35/00, A61P 43/00, A61N 5/10

(54) **USE OF DNASE TO IMPROVE SAFETY AND EFFICACY OF CANCER RADIOTHERAPY**
VERWENDUNG VON DNASE ZUR VERBESSERUNG DER WIRKSAMKEIT UND SICHERHEITSPROFIL DER STRAHLENTHERAPIE GEGEN KREBS
UTILISATION DE LA DNASE POUR AMÉLIORER L'INNOCUITÉ ET L'EFFICACITÉ DE LA RADIOTHÉRAPIE ANTICANCÉREUSE

(43) Date of publication of application: 15.09.2021
(62) Divisional of application: 15907392.3
(73) Proprietor: CLS Therapeutics Limited, Guernsey, Channel Islands, GY1 1GX (GG)
(72) Inventor: GENKIN, Dmitry Dmitrievich, 197110 St. Petersburg (RU); TETS, Georgy Viktorovich, 191040 St. Petersburg (RU); TETS, Viktor Veniaminovich, 196066 St. Petersburg (RU)
(74) Representative: Grund, Martin

(56) References cited:
- EP-A1- 1 666 055
- WO-A2-01/82949
- RU-C2- 2 269 359
- TRESHALIN I D ET AL: "MODIFICATION OF ANTITUMOR DRUGS TOXICITY AS A METHOD OF ENHAN cing anticancer chemotherapeutic efficacy>", ROSSIISKII BIOTERAPEVTICHESKII ZHURNAL, GU RONTS IM. N. N. BLOKHINA RAMN, RUSSIA, vol. 4, no. 3, 1 January 2005 (2005-01-01), pages 87 - 94, XP009505075, ISSN: 1726-9784
- M. C. HAWES ET AL: "Extracellular DNA: A Bridge to Cancer", CANCER RESEARCH, vol. 75, no. 20, 15 October 2015 (2015-10-15), US, pages 4260 - 4264, XP055379708, ISSN: 0008-5472, DOI: 10.1158/0008-5472.CAN-15-1546
- DATABASE EMBASE [online] ELSEVIER SCIENCE PUBLISHERS, AMSTERDAM, NL; 1972, ESPOSITO S ET AL: "The place of desoxyribonuclease in the treatment of chronic lymphatic leukemia", XP002791163, Database accession no. EMB-1974079505
- MITTAL BHARAT B ET AL: "Effect of Recombinant Human Deoxyribonuclease on Oropharyngeal Secretions in Patients With Head-and-Neck Cancers Treated With Radiochemotherapy", INTERNATIONAL JOURNAL OF RADIATION ONCOLOGY BIOLOGY PHYSICS, vol. 87, no. 2, 1 October 2013 (2013-10-01), pages 282 - 289, XP002791164
- ANONYMOUS: "Route of administration - Wikipedia", 17 November 2022 (2022-11-17), XP093009054, Retrieved from the Internet <URL:https://en.wikipedia.org/wiki/Route_of_administration> [retrieved on 20221216]

## Description

### FIELD OF THE INVENTION

The invention relates to a deoxyribonuclease (DNase) enzyme for use in preventing or ameliorating at least one side effect of radiation therapy in a subject suffering from a cancer and receiving said radiation therapy, by preventing or ameliorating a toxicity associated with said radiation therapy, wherein the DNase enzyme is to be administered parenterally.

### BACKGROUND OF THE INVENTION

Cancers are a very significant cause of death in humans. The leading cancer therapies today are surgery, radiation and cytostatic and/or cytotoxic chemotherapy. Despite of advances in the field of chemotherapy treatments, most of the known chemotherapies are associated with serious side effects including myelopathy, hepatopathy, digestive disorders (e.g., nausea, vomiting, anorexia, diarrhea, constipation), pulmonary insufficiency, dermatopathy, nervous system disorders, endocrine disorders, genital disorders, cardiovascular disorders, hepatopathy, pancreatic disorder, nephropathy, bladder trouble, hyperuricemia, decrease of immunocompetence, infections, hypersensitivity to light, hair loss, etc. (2-5). These side effects are life threatening or seriously debilitating and cause significant chemotherapy-related morbidity and mortality.

One of the major complications of cancer chemotherapy is damage to bone marrow cells or suppression of their function. Specifically, chemotherapy damages or destroys hematopoietic precursor cells, primarily found in the bone marrow and spleen, impairing the production of new blood cells (granulocytes, lymphocytes, erythrocytes, monocytes, platelets, etc.). Many cancer patients die of infection or other consequences of hematopoietic failure subsequent to chemotherapy. Chemotherapeutic agents can also result in subnormal formation of platelets, which produces a propensity toward hemorrhage. Inhibition of erythrocyte production can result in anemia. It has been also recently recognized that development of more potent cytotoxic therapies and more effective chemotherapy regimens for a wider range of malignancies significantly increases the frequency of a serious toxic adverse event termed Tumor Lysis Syndrome (TLS) (16). TLS is a group of metabolic complications that can occur as a result of administration of cytotoxic therapies, most often in the context of chemotherapy for lymphomas and leukemias, and are caused by the breakdown products of dying cells.

The main reason chemotherapy is so debilitating and the symptoms so severe is that chemotherapeutic drugs are often unable to differentiate between normal, healthy cells and tumor cells they are designed to target. Another mechanism responsible for chemotherapy-related toxicity is toxic effects of cellular components released from cells undergoing necrosis or apoptosis as result of chemotherapy-induced cell death.

The side effects associated with chemotherapeutic drugs limit the frequency and dosage at which such drugs can be administered leading to lower efficacy.

As the concept of systemic cytotoxic chemotherapy has evolved, a lot of research efforts have been spent to identify possible approaches to attenuate chemotherapy-related toxicity and avoid the cessation of the patients' exposure to chemotherapeutic agents. Chemotherapeutic dosing and schedule modulation leading to the development of less toxic dosing modalities is one possibility (6). Others have proposed dietary approaches, such as fasting or restricting specific nutrients during and after chemotherapy (7); and supplementation of the patient's diet with several specific dietary amino acids (8). The use of certain drug-specific metabolic antidotes, particularly acylated derivatives of uridine or cytidine, for the prevention of toxicity induced by pyrimidine nucleoside analogs is disclosed in U.S. Patent No. 7,776,838. Use of chromanol glycoside for prevention of toxicity induced by alkylating agents is disclosed in U.S. Patent No. 7,462,601. Use of antioxidants for prevention of anthracycline-induced cardiotoxicity has also been proposed (9). Attenuation of tissue-specific toxicity of chemotherapeutic drugs, in particular prevention of mucositis using topical application of alpha-interferon or beta-interferon (U.S. Patent No. 5,017,371); prevention of stomach and bowel side effect using selective glucagon-like-peptide-2 (GLP-2) analogues (U.S. Patent No. 8,642,727); prevention of liver damage using A2B adenosine receptor antagonist (U.S. Patent No. 8,188,099), and prevention of prostate damage using inhibitor of IGFBP-1 growth factor (U.S. Patent No. 8,211,700) has been also disclosed. Use of alkaline phosphatase enzyme to ameliorate general toxicity of chemotherapy and to maintain healthy muscle and adipose tissue mass in mammals receiving chemotherapy has been disclosed in U.S. Patent No. 8,460,654.

Development of new compositions and methods for preventing or ameliorating chemotherapy-related toxicity is highly desired.

Current radiotherapies for treatment of cancers provide significant benefits for patients with early stage and radiosensitive cancers, but these benefits are much less significant for patients with radioresistant tumors (e.g., brain or pancreas cancers) and for patients with late stage tumors. For these patients, the radiation needed to eradicate the tumor can cause intolerable or fatal radiation damage. This is especially the case for pediatric patients, whose rapidly developing normal tissues are often more radiosensitive than their tumors, and who therefore cannot tolerate radiotherapy that would be curative for adults with the same disease. Damage to normal tissue limits the use of radiotherapy treatments for cancer patients of a young age, patients with central nerve system cancers, radioresistant cancers, and late stage cancer with large tumors.

Development of new compositions and methods for preventing or ameliorating radiotherapy-related toxicity is highly desired.

### SUMMARY OF THE INVENTION

As specified in the Background Section, there is a great need in the art to develop new compositions and methods for preventing or ameliorating chemotherapy- and radiotherapy-related toxicity.

As stated above, the invention relates to a deoxyribonuclease (DNase) enzyme for use in preventing or ameliorating at least one side effect of radiation therapy in a subject suffering from a cancer and receiving said radiation therapy, by preventing or ameliorating a toxicity associated with said radiation therapy, wherein the DNase enzyme is to be administered parenterally.

One aspect of the present disclosure is a method for preventing or ameliorating a toxicity associated with a cytostatic and/or cytotoxic chemotherapy in a subject suffering from a cancer and receiving or deemed to receive said chemotherapy, which method comprises administering to the subject a therapeutically effective amount of a DNase enzyme, wherein said amount of the DNase enzyme is effective to prevent or ameliorate at least one side effect of said chemotherapy. Another aspect of the present disclosure is a method for increasing efficacy of a cytostatic and/or cytotoxic chemotherapy in a subject suffering from a cancer and receiving or deemed to receive said chemotherapy, which method comprises administering to the subject a therapeutically effective amount of a DNase enzyme, wherein said amount of the DNase enzyme is effective to prevent or ameliorate at least one side effect of said chemotherapy and wherein the DNase administration results in prevention or amelioration of toxicity associated with said chemotherapy.

In one specific variation of the above methods, said side effect of said chemotherapy is selected from the group consisting of body weight loss, bone marrow toxicity, catabolic changes in blood biochemistry, myocardial necrosis, gastrointestinal toxicity, suppression of immunity, and neutropenia.

A further aspect of the disclosure is a method for preventing or ameliorating a catabolic state leading to body weight loss associated with a cytostatic and/or cytotoxic chemotherapy in a subject suffering from a cancer and receiving or deemed to receive said chemotherapy, which method comprises administering to the subject a therapeutically effective amount of a DNase enzyme, wherein said amount of the DNase enzyme is effective to prevent or ameliorate the catabolic state leading to body weight loss associated with said chemotherapy. In one specific variation, the chemotherapy is anthracycline-containing therapy.

Yet another aspect of the present disclosure is a method for preventing or ameliorating a bone marrow toxicity and/or catabolic changes in blood biochemistry associated with a cytostatic and/or cytotoxic chemotherapy in a subject suffering from a cancer and receiving or deemed to receive said chemotherapy, which method comprises administering to the subject a therapeutically effective amount of a DNase enzyme, wherein said amount of the DNase enzyme is effective to prevent or ameliorate the bone marrow toxicity and/or catabolic changes in blood biochemistry associated with said chemotherapy. In one specific variation, the chemotherapy is anthracycline-containing therapy.

A further aspect of the present disclosure is a method for preventing or ameliorating a cardiotoxicity associated with a cytostatic and/or cytotoxic chemotherapy in a subject suffering from a cancer and receiving or deemed to receive said chemotherapy, which method comprises administering to the subject a therapeutically effective amount of a DNase enzyme, wherein said amount of the DNase enzyme is effective to prevent or ameliorate the cardiotoxicity associated with said chemotherapy. In one specific variation, the cardiotoxicity is myocardial necrosis. In one specific variation, the chemotherapy is anthracycline-containing therapy.

Another aspect of the present disclosure is a method for preventing or ameliorating a gastrointestinal toxicity associated with a cytostatic and/or cytotoxic chemotherapy in a subject suffering from a cancer and receiving or deemed to receive said chemotherapy, which method comprises administering to the subject a therapeutically effective amount of a DNase enzyme, wherein said amount of the DNase enzyme is effective to prevent or ameliorate the gastrointestinal toxicity associated with said chemotherapy. In one specific variation, the chemotherapy is 5-fluouracil- and/or etoposide-containing therapy.

Yet another aspect of the present disclosure is a method for preventing or ameliorating a suppression of immunity associated with a cytostatic and/or cytotoxic chemotherapy in a subject suffering from a cancer and receiving or deemed to receive said chemotherapy, which method comprises administering to the subject a therapeutically effective amount of a DNase enzyme, wherein said amount of the DNase enzyme is effective to prevent or ameliorate the suppression of immunity associated with said chemotherapy. In one specific variation, the chemotherapy is taxane-containing therapy.

A further aspect of the present disclosure is a method for preventing or ameliorating neutropenia associated with a cytostatic and/or cytotoxic chemotherapy in a subject suffering from a cancer and receiving or deemed to receive said chemotherapy, which method comprises administering to the subject a therapeutically effective amount of a DNase enzyme, wherein said amount of the DNase enzyme is effective to prevent or ameliorate neutropenia associated with said chemotherapy. In one specific variation, the chemotherapy is cyclophosphamide-containing therapy.

In one variation of any of the above methods, the chemotherapy comprises administration of one or more compounds selected from the group consisting of antimetabolites, alkylating agents, anticancer antibiotics, microtubule-targeting agents, topoisomerase inhibitors, alkaloids, and targeted therapeutics.

In one variation of any of the above methods, the chemotherapy comprises administration of one or more compounds selected from the group consisting of anthracycline, doxorubicin, 5-fluorouracil (5-FU), etoposide, taxane, and cyclophosphamide.

In one variation of any of the above methods, the DNase enzyme is administered during a cycle of the chemotherapy. In another variation of any of the above methods, the DNase enzyme is administered after a cycle of the chemotherapy.

Another aspect of the present disclosure is a method for preventing or ameliorating a toxicity associated with a radiation therapy in a subject suffering from a cancer and receiving said radiation therapy, which method comprises administering to the subject a therapeutically effective amount of a DNase enzyme, wherein said amount of the DNase enzyme is effective to prevent or ameliorate at least one side effect of said radiation therapy.

A further aspect of the present disclosure is a method for increasing the efficacy of a radiation therapy in a subject suffering from a cancer and receiving said radiation therapy, which method comprises administering to the subject a therapeutically effective amount of a DNase enzyme, wherein said amount of the DNase enzyme is effective to prevent or ameliorate at least one side effect of said radiation therapy and wherein the DNase administration results in prevention or amelioration of toxicity associated with said radiation therapy.

In one variation of the above two methods, the side effect of the radiation therapy is selected from the group consisting of skin irritation or damage, fatigue, nausea, vomiting, fibrosis, bowel damage, memory loss, infertility, and a second cancer.

Yet another aspect of the present disclosure is a method for preventing or ameliorating a body weight loss associated with a radiation therapy in a subject suffering from a cancer and receiving said radiation therapy, which method comprises administering to the subject a therapeutically effective amount of a DNase enzyme, wherein said amount of the DNase enzyme is effective to prevent or ameliorate body weight loss associated with said radiation therapy.

In one variation of the above three methods, the radiation therapy is external beam radiation therapy or systemic radioisotope therapy.

In one variation of the above three methods, the DNase enzyme is administered during a cycle of the radiation therapy. In another variation, the DNase enzyme is administered after a cycle of the radiation therapy.

In one variation of any of the above methods, the DNase enzyme is DNase I (e.g., human recombinant DNase I or bovine pancreatic DNase I) or an analogue thereof (e.g., DNase X, DNase gamma, or DNAS1L2). In another variation of any of the above methods, the DNase enzyme is DNase II. In one embodiment, the DNase enzyme has an extended half-life (e.g., is conjugated with polysialic acid or is protected from binding to actin by modification of actin binding-site).

In one variation of any of the above methods, the therapeutically effective amount of a DNase enzyme is at least 0.5 mg/kg/day or at least 1000 Kunitz units (KU)/kg/day, preferably at least 1.5 mg/kg/day or at least 3000 KU/kg/day. In one variation of any of the above methods, the therapeutically effective amount of a DNase enzyme is from 0.5 to 100 mg/kg/day or from 1000 to 200000 KU/kg/day, preferably from 0.5 to 50 mg/kg/day or from 1000 to 100000 KU/kg/day, more preferably from 1.5 to 50 mg/kg/day or from 3000 to 100000 KU/kg/day, most preferably from 10 to 50 mg/kg/day or from 20000 to 100000 KU/kg/day.

In one variation of any of the above methods, the DNase enzyme is administered intravenously or intraperitoneally. In one variation of any of the above methods, the DNase enzyme is administered enterally (e.g., orally).

In one variation of any of the above methods, the subject is human.

These and other aspects of the present invention will be apparent to those of ordinary skill in the art in the following description, claims and drawings.

### BRIEF DESCRIPTION OF THE DRAWINGS

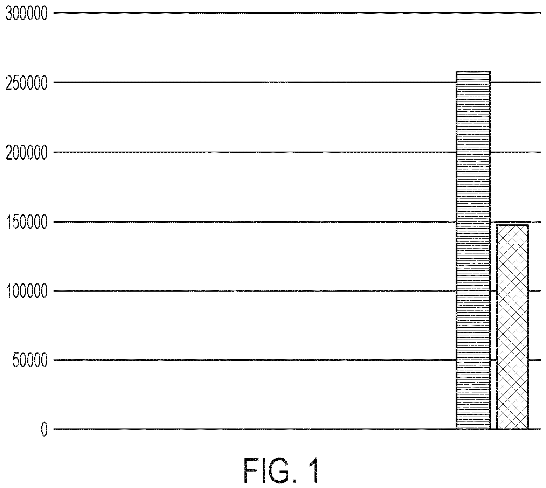

**Figure 1** is a bar graph showing myocardial necrosis area in deceased rats challenged with sub-lethal IV doses of doxorubicin (7.5 mg/kg) and either daily IP injections of human recombinant DNase I (15 mg/kg) (black bar) or daily IP injections of placebo (WFI) (diagonal hatched bar). Serial myocardium microscopy samples from each heart were analyzed using an automated video analyzer to quantify the necrotic areas. The sum of necrotic area (Sₙₐ; nm²) was calculated as a sum of necrotic areas in 30 serial slides from each individual autopsied heart (n=3 for DNase I-treated rats - black bar; n=5 for placebo-treated rats - diagonal hatched bar).

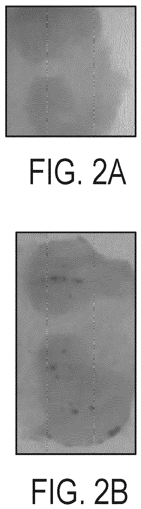

**Figures 2A-B** show the photographs of stained stomachs of rats treated with oral Etoposide (200 mg/kg) and 5-fluorouracil (5-FU; 400 mg/kg) followed either by (A) IV treatment with human recombinant DNase I (50 mg/kg) or by (B) IV placebo (WFI). Multiple erosions and ulcers are visible in the stomach in panel (B), while the stomach in panel (A) does not have any macroscopic abnormalities.

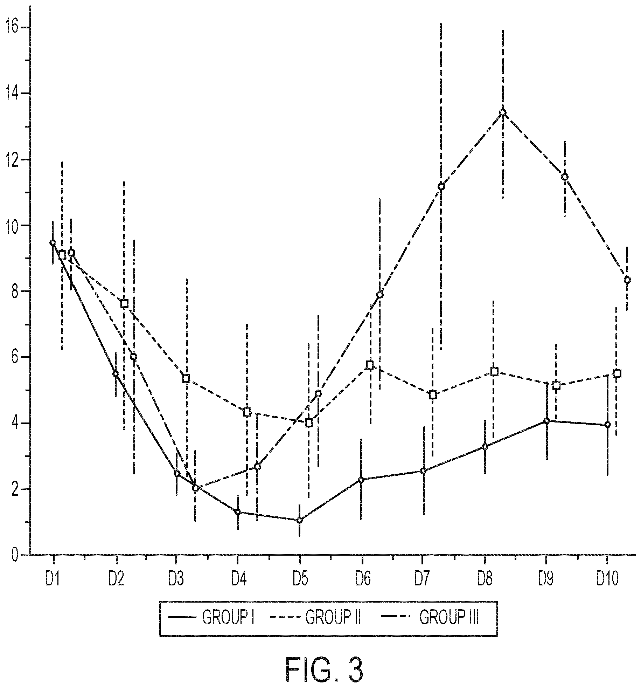

**Figure 3** is a graph summarizing the dynamics of the white blood cell count (WBC) in the three experimental groups, in which neutropenia was induced by a single IP injection of cyclophosphamide (200 mg/kg) followed by no treatment (Group I) or IV treatment with human recombinant DNase I (25 mg/kg) (Group II) or SC treatment with human recombinant GM-CSF (200 pg/kg) (Group III). The vertical axis represents WBC with units of 10⁹ WBC/L. The horizontal axis represents the number of days after cyclophosphamide injection. The figure demonstrates the ameliorating effect of DNase treatment on neutropenia induced by an alkylating agent such as cyclophosphamide.

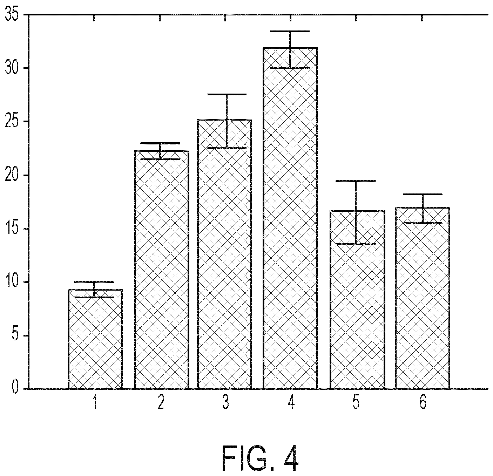

**Figure 4** is a bar graph showing a summary of survival data for animals transplanted with L1210 leukemia cells to induce cancer followed by treatment with cytosine arabinoside (AraC) and/or DNase II. The six experimental groups are represented on the horizontal axis: 1. Negative Control; 2. AraC (positive control); 3. AraC + DNase II (15 mg/kg); 4. AraC + DNase II (45 mg/kg); 5. DNase II (15 mg/kg); 6. DNase II (45 mg/kg). The figure demonstrates the synergistic effects of DNase and AraC treatment on survival rates of mice with leukemia.

### DETAILED DESCRIPTION OF THE INVENTION

The present inventors have previously demonstrated that deoxyribonuclease (DNase) enzyme is a useful agent for treating cancer (see, e.g., U.S. Patent Appl. Pub. No. 2010/0150903 and U.S. Patent No. 7,612,032), a property which has been recently confirmed by several authors (10-12). However, it has been also reported that DNase lacks a significant influence on tolerability of antiproliferative chemotherapy (15) and can even significantly contribute to increase in toxicity of chemotherapy (17).

The present invention is based on an unexpected discovery by the inventors that DNase enzymes not only increase efficacy of cytostatic and/or cytotoxic chemotherapy, but also significantly decrease the chemotherapy-related toxicity. This discovery is especially surprising in light of prior reports on lack of DNase influence on tolerability of chemotherapy (15) and DNase contribution to increase in nephrotoxicity of chemotherapy (17). The ability of DNase to prevent and/or ameliorate chemotherapy-related toxicity is shared by different types of DNase enzymes and is drug- and tissue-independent making DNases highly desirable and potent candidates for chemotherapy accessory therapy. Indeed, as demonstrated in the Examples section, below, various types of DNase enzymes (e.g., DNase I, DNase gamma, and DNase II) can be used to ameliorate toxicity of such diverse types of chemotherapeutic agents as Doxorubicin (anthracycline antibiotic which works by intercalating DNA), 5-FU (antimetabolite which works through inhibition of thymidylate synthase), Etoposide (topoisomerase inhibitor), Taxane (microtubule disruptor), and Cyclophosphamide (alkylating agent).

### Definitions

The terms "cytostatic and/or cytotoxic chemotherapy" and "chemotherapy" are used interchangeably herein to refer to a therapy involving administering of a cytostatic and/or cytotoxic agent.

The terms "anti-cancer agent" and "anti-cancer chemotherapeutic agent" are used herein to refer to any chemical compound, which is used to treat cancer. Anti-cancer chemotherapeutic agents are well known in the art (see, e.g., Gilman A. G., et al., The Pharmacological Basis of Therapeutics, 8th Ed., Sec 12:1202-1263 (1990)). Specific examples of chemotherapeutic agents are provided throughout the specification.

The term "side effect of a chemotherapy" as used herein refers to an undesirable and unintended, although not necessarily unexpected, result of a chemotherapy.

As used herein, the terms "radiotherapy", "radiation therapy", and "RT" are used interchangeably to refer to the medical use of ionizing radiation as part of a cancer treatment to damage the DNA of malignant cells, either directly or by creating charged particles within the affected cells that damage the DNA. Commonly used types of radiation therapy encompassed by the present invention include, e.g., external beam radiation therapy (EBRT or XRT), brachytherapy/sealed source radiation therapy, and systemic radioisotope therapy/unsealed source radiotherapy

The terms "side effect of a radiotherapy" or "side effect of a radiation therapy" as used herein refer to an undesirable and unintended, although not necessarily unexpected, result of a radiation therapy. Which side effects develop depend on the area of the body being treated, the dose given per day, the total dose given, the patient's general medical condition, and other treatments given at the same time, and may include, e.g., skin irritation or damage, fatigue, nausea, vomiting, fibrosis, bowel damage, memory loss, infertility, or a second cancer.

The term "catabolic state" as used herein refers to a condition characterized by a rapid weight loss and loss of fat and skeletal muscle mass, which may occur in a background of chemotherapy or chemoradiation therapy. Associated clinical events include, for example, immunosuppression, muscle weakness, predisposition to pulmonary embolism, thrombophlebitis, and altered stress response.

As used herein, the terms "deoxyribonuclease" and "DNase" are used to refer to any enzyme that catalyzes the hydrolytic cleavage of phosphodiester linkages in the DNA backbone. A wide variety of deoxyribonucleases is known and can be used in the methods of the present disclosure. Non-limiting examples of DNases useful in the methods of the present disclosure include, e.g., DNase I (e.g., human recombinant DNase I or bovine pancreatic DNase I), analogues of DNase I (such as, e.g., DNase X, DNase gamma, and DNAS1L2), DNase II, phosphodiesterase I, lactoferrin, and acetylcholinesterase. Also encompassed by the present invention are DNase enzymes which have an extended half-life (e.g., DNase enzymes conjugated with polysialic acid or protected from binding to actin by modification of actin binding-site; see, e.g., Gibson et al., (1992) J. Immunol. Methods, 155, 249-256). DNase I cleaves DNA preferentially at phosphodiester linkages adjacent to a pyrimidine nucleotide, yielding 5'5'-phosphate-terminated polynucleotides with a free hydroxyl group on position 3', on average producing tetranucleotides. DNase I acts on single-stranded DNA, double-stranded DNA, and chromatin.

The term "about" means within an acceptable error range for the particular value as determined by one of ordinary skill in the art, which will depend in part on how the value is measured or determined, i.e., the limitations of the measurement system. For example, "about" can mean within an acceptable standard deviation, per the practice in the art. Alternatively, "about" can mean a range of up to +20%, preferably up to +10%, more preferably up to +5%, and more preferably still up to +1% of a given value. Alternatively, particularly with respect to biological systems or processes, the term can mean within an order of magnitude, preferably within 2-fold, of a value. Where particular values are described in the application and claims, unless otherwise stated, the term "about" is implicit and in this context means within an acceptable error range for the particular value.

In the context of the present invention insofar as it relates to any of the disease conditions recited herein, the terms "treat", "treatment", and the like mean to relieve or alleviate at least one symptom associated with such condition, or to slow or reverse the progression of such condition. Within the meaning of the present invention, the term "treat" also denotes to arrest, delay the onset (i.e., the period prior to clinical manifestation of a disease) and/or reduce the risk of developing or worsening a disease. For example, in connection with cancer the term "treat" may mean eliminate or reduce a patient's tumor burden, or prevent, delay or inhibit metastasis, etc.

As used herein the term "therapeutically effective" applied to dose or amount refers to that quantity of a compound or pharmaceutical composition that is sufficient to result in a desired activity upon administration to a subject in need thereof. Within the context of the present invention, when the term "therapeutically effective" is used in connection with the use of deoxyribonuclease (DNase) to ameliorate or prevent side effects of a cytostatic and/or cytotoxic chemotherapy, it refers to an amount of DNase or a pharmaceutical composition containing DNase that is effective to ameliorate or prevent at least one side effect of such cytostatic and/or cytotoxic chemotherapy. Note that when a combination of active ingredients is administered (e.g., a combination of DNase and another compound effective for ameliorating or preventing side effects of chemotherapy) the effective amount of the combination may or may not include amounts of each ingredient that would have been effective if administered individually.

The phrase "pharmaceutically acceptable", as used in connection with compositions disclosed herein, refers to molecular entities and other ingredients of such compositions that are physiologically tolerable and do not typically produce untoward reactions when administered to a subject (e.g., a mammal such as a human). Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans.

The term "targeted therapeutics" is used to refer to a class of chemical and biological compounds that may be effective in patients whose cancers have a specific molecular target, but they may not be effective in the absence of such a target (18).

As used herein, the term "subject" refers to any mammal. In a preferred embodiment, the subject is human.

As used in this specification and the appended claims, the singular forms "a," "an," and "the" include plural references unless the context clearly dictates otherwise.

In accordance with the present invention there may be employed conventional pharmacology and molecular biology techniques within the skill of the art. Such techniques are explained fully in the literature. See, *e.g.,* Sambrook, Fritsch & Maniatis, Molecular Cloning: A Laboratory Manual, Second Edition (1989) Cold Spring Harbor Laboratory Press, Cold Spring Harbor, New York (herein "Sambrook et al., 1989"); DNA Cloning: A Practical Approach, Volumes I and II (D.N. Glover ed. 1985); Oligonucleotide Synthesis (MJ. Gait ed. 1984); Nucleic Acid Hybridization (B.D. Hames & S.J. Higgins eds. (1985)); Transcription and Translation (B.D. Hames & S.J. Higgins, eds. (1984)); Animal Cell Culture (R.I. Freshney, ed. (1986)); Immobilised Cells and Enzymes (IRL Press, (1986)); B. Perbal, A Practical Guide To Molecular Cloning (1984); F.M. Ausubel et al. (eds.), Current Protocols in Molecular Biology, John Wiley & Sons, Inc. (1994); among others.

### Therapeutic Methods

One aspect of the present disclosure is a method for preventing or ameliorating a toxicity associated with a cytostatic and/or cytotoxic chemotherapy in a subject suffering from a cancer and receiving or deemed to receive said chemotherapy, which method comprises administering to the subject a therapeutically effective amount of a DNase enzyme (e.g., DNase I (e.g., human recombinant DNase I or bovine pancreatic DNase I), analogues of DNase I (such as, e.g., DNase X, DNase gamma, or DNAS1L2), DNase II, phosphodiesterase I, lactoferrin, or acetylcholinesterase), wherein said amount of the DNase enzyme is effective to prevent or ameliorate at least one side effect of said chemotherapy.

A further aspect of the present disclosure is a method for increasing the efficacy of a cytostatic and/or cytotoxic chemotherapy in a subject suffering from a cancer and receiving or deemed to receive said chemotherapy, which method comprises administering to the subject a therapeutically effective amount of a DNase enzyme (e.g., DNase I (e.g., human recombinant DNase I or bovine pancreatic DNase I), analogues of DNase I (such as, e.g., DNase X, DNase gamma, or DNAS1L2), DNase II, phosphodiesterase I, lactoferrin, or acetylcholinesterase), wherein said amount of the DNase enzyme is effective to prevent or ameliorate at least one side effect of said chemotherapy and wherein the DNase administration results in prevention or amelioration of toxicity associated with said chemotherapy.

DNase enzymes can be used to ameliorate toxicity of a wide range of different chemotherapeutic agents. Non-limiting examples of such agents include anti-metabolites such as pyrimidine analogs (e.g., 5-fluorouracil [5-FU], floxuridine, capecitabine, gemcitabine and cytarabine) and purine analogs, folate antagonists and related inhibitors (e.g., mercaptopurine, thioguanine, pentostatin and 2-chlorodeoxyadenosine (cladribine)); antiproliferative/antimitotic agents including natural products such as vinca alkaloids (e.g., vinblastine, vincristine, and vinorelbine), microtubule disruptors such as taxanes (e.g., paclitaxel, docetaxel), vincristin, vinblastin, nocodazole, epothilones and navelbine, epidipodophyllotoxins (e.g., etoposide, teniposide), DNA damaging agents (e.g., actinomycin, amsacrine, anthracyclines, bleomycin, busulfan, camptothecin, carboplatin, chlorambucil, cisplatin, nedaplatin, cyclophosphamide, cytoxan, dactinomycin, daunorubicin, doxorubicin, epirubicin, aclarubicin, purarubicin, hexamethyhnelamineoxaliplatin, iphosphamide, melphalan, merchlorehtamine, mitomycin, mitoxantrone, nitrosourea, nimustine, ranimustine, estramustine, plicamycin, procarbazine, taxol, taxotere, teniposide, triethylenethiophosphoramide and etoposide (VP16)); antibiotics (e.g., dactinomycin (actinomycin D), daunorubicin, doxorubicin (adriamycin), idarubicin, anthracyclines, mitoxantrone, bleomycins, plicamycin (mithramycin), pleomycin, peplomycin, mitomycins (e.g., mitomycin C), actinomycins (e.g., actinomycin D), zinostatinstimalamer); enzymes (e.g., L-asparaginase); neocarzinostatin; antiplatelet agents; antiproliferative/antimitotic alkylating agents such as nitrogen mustards (e.g., mechlorethamine, cyclophosphamide and analogs, imidazol carboxamide, melphalan, chlorambucil, nitrogen mustard-N-oxide hydrochloride, ifosfamide), ethylenimines and methylmelamines (e.g., hexamethylmelamine, thiotepa, carboquone, triethylene thiophospharamide), alkyl sulfonates (e.g., busulfan, isoprosulfan tosylate), nitrosoureas (e.g., carmustine (BCNU) and analogs, streptozocin), trazenes-dacarbazinine (DTIC); epoxide type compounds (e.g., mitobronitol); antiproliferative/antimitotic antimetabolites such as folic acid analogs (e.g., methotrexate); platinum coordination complexes (e.g., cisplatin, carboplatin), procarbazine, hydroxyurea, mitotane, aminoglutethimide; hormones, hormone analogs (e.g., estrogen, tamoxifen, goserelin, bicalutamide, nilutamide) and aromatase inhibitors (e.g., letrozole, anastrozole); antisecretory agents (e.g., breveldin); immunosuppressives (e.g., cyclosporine, tacrolimus (FK-506), sirolimus (rapamycin), azathioprine, mycophenolate mofetil); anti-angiogenic compounds (e.g., TNP-470, genistein, bevacizumab) and growth factor inhibitors (e.g., fibroblast growth factor (FGF) inhibitors); angiotensin receptor blockers; nitric oxide donors; antisense oligonucleotides; antibodies (e.g., trastuzumab); cell cycle inhibitors and differentiation inducers (e.g., tretinoin); mTOR inhibitors, topoisomerase inhibitors (e.g., doxorubicin (adriamycin), amsacrine, camptothecin, daunorubicin, dactinomycin, eniposide, epirubicin, etoposide, idarubicin, mitoxantrone, topotecan, irinotecan); growth factor signal transduction kinase inhibitors; mitochondrial dysfunction inducers; chromatin disruptors; sobuzoxane; tretinoin; pentostatin; flutamide; porphimer natrium; fadrozole; procarbazine; aceglatone; radioimmunotherapy (RIT) compounds (e.g., Ibritumomab tiuxetan, Iodine (¹³¹I) tositumomab); and targeted radionuclide therapy (TRT) compounds (e.g., samarium-153-EDTMP, strontium-89-chloride).

Non-limiting examples of side effects of cytostatic and/or cytotoxic chemotherapy which can be prevented or ameliorated by administering DNase enzymes according to the methods disclosed herein include, for example, bone marrow toxicity, neutropenia, myelopathy (e.g., leukopenia, granulocytopenia, lymphopenia, thrombocytopenia, erythropenia); hematopathy (e.g., plasma fibrinogenopenia); catabolic changes in blood biochemistry; gastrointestinal disorders (e.g., nausea, vomiting, anorexia, body weight loss, heavy feeling of stomach, diarrhea, constipation, stomatitis, esophagitis); pulmonary insufficiency (e.g., chronic pneumonia, lung fibrosis, ARDS, ALS, lung emboli); dermatopathy (e.g., keratinization, pachymenia, chromatosis, epilation, rash, nail alternation, cancer-induced alopecia); nervous system disorders (e.g., paresthesia, depression, deep areflexia, neuroparalysis, auditory disorder, allolalia, disorientation, neurologic manifestation, cerebellar ataxia, somnolence, coma, vertigo, frequency of micturition, frequency of defecation desire); endocrine disorders (e.g., pituitary disorder, adrenal disorder, hyperglycemia, hypoglycemia); genital disorders (e.g., hyposexuality, oligospermia, gynecomastia, menstrual disorder); cardiovascular disorders (e.g., myocardial necrosis, cardiomyopathy, arrhythmia, low blood pressure, tachycardia, cardiac failure); hepatopathy, pancreatic disorder, nephropathy, bladder trouble, hyperuricemia, decrease of immunocompetence, and infection.

Another aspect of the present disclosure is a method for preventing or ameliorating a toxicity associated with a radiation therapy in a subject suffering from a cancer and receiving said radiation therapy, which method comprises administering to the subject a therapeutically effective amount of a DNase enzyme (e.g., DNase I (e.g., human recombinant DNase I or bovine pancreatic DNase I), analogues of DNase I (such as, e.g., DNase X, DNase gamma, or DNAS1L2), DNase II, phosphodiesterase I, lactoferrin, or acetylcholinesterase), wherein said amount of the DNase enzyme is effective to prevent or ameliorate at least one side effect of said radiation therapy.

A further aspect of the present disclosure is a method for increasing the efficacy of a radiation therapy in a subject suffering from a cancer and receiving said radiation therapy, which method comprises administering to the subject a therapeutically effective amount of a DNase enzyme (e.g., DNase I (e.g., human recombinant DNase I or bovine pancreatic DNase I), analogues of DNase I (such as, e.g., DNase X, DNase gamma, or DNAS1L2), DNase II, phosphodiesterase I, lactoferrin, or acetylcholinesterase), wherein said amount of the DNase enzyme is effective to prevent or ameliorate at least one side effect of said radiation therapy and wherein the DNase administration results in prevention or amelioration of toxicity associated with said radiation therapy.

According to the present invention, DNase enzymes can be used to ameliorate toxicity and increase efficacy of various types of radiation therapy, including, for example, external beam radiation therapy (EBRT or XRT), brachytherapy/sealed source radiation therapy, and systemic radioisotope therapy/unsealed source radiotherapy.

Non-limiting examples of side effects of radiotherapy which can be prevented or ameliorated by administering DNase enzymes according to the methods disclosed herein include, for example, skin irritation or damage, fatigue, nausea, vomiting, fibrosis, bowel damage, memory loss, infertility, and a second cancer.

The methods disclosed herein can be used in subjects suffering from a broad range of cancers, which subjects are subjected to anti-cancer chemotherapeutic treatments which result in deleterious side effects. Non-limiting examples of relevant cancers include, e.g., breast cancer, prostate cancer, multiple myeloma, transitional cell carcinoma, lung cancer (e.g., non-small cell lung cancer (NSCLC)), renal cancer, thyroid cancer, leukemia (e.g., chronic myeloid leukemia, acute myeloid leukemia, chronic lymphocytic leukemia, acute lymphocytic leukemia), lymphoma (e.g., B cell lymphoma, T cell lymphoma, non-Hodgkins lymphoma, Hodgkins lymphoma), head and neck cancer, esophageal cancer, stomach cancer, colon cancer, intestinal cancer, colorectal cancer, rectal cancer, pancreatic cancer, liver cancer, cancer of the bile duct, cancer of the gall bladder, ovarian cancer, uterine endometrial cancer, vaginal cancer, cervical cancer, bladder cancer, neuroblastoma, sarcoma, osteosarcoma, malignant melanoma, squamous cell cancer, bone cancer, including both primary bone cancers (e.g., osteosarcoma, chondrosarcoma, Ewing's sarcoma, fibrosarcoma, malignant fibrous histiocytoma, adamantinoma, giant cell tumor, and chordoma) and secondary (metastatic) bone cancers, soft tissue sarcoma, basal cell carcinoma, angiosarcoma, hemangiosarcoma, myxosarcoma, liposarcoma, osteogenic sarcoma, angiosarcoma, endotheliosarcoma, lymphangiosarcoma, lymphangioendotheliosarcoma, synovioma, testicular cancer, uterine cancer, gastrointestinal cancer, mesothelioma, leiomyosarcoma, rhabdomyosarcoma, adenocarcinoma, sweat gland carcinoma, sebaceous gland carcinoma, papillary carcinoma, Waldenstroom's macroglobulinemia, papillary adenocarcinomas, cystadenocarcinoma, bronchogenic carcinoma, choriocarcinoma, seminoma, embryonal carcinoma, Wilms' tumor, epithelial carcinoma, glioma, glioblastoma, astrocytoma, medulloblastoma, craniopharyngioma, ependymoma, pinealoma, hemangioblastoma, acoustic neuroma, oligodendroglioma, meningioma, retinoblastoma, medullary carcinoma, thymoma, sarcoma, etc.

In one variation, a method is disclosed for preventing or ameliorating a catabolic state leading to body weight loss associated with a cytostatic and/or cytotoxic chemotherapy (e g , doxorubicin therapy) in a subject suffering from a cancer and receiving or deemed to receive said chemotherapy, which method comprises administering to the subject a therapeutically effective amount of a DNase enzyme (e g., DNase I (e g., human recombinant DNase I or bovine pancreatic DNase I), analogues of DNase I (such as, e g , DNase X, DNase gamma, or DNAS1L2), DNase II, phosphodiesterase I, lactoferrin, or acetylcholinesterase), wherein said amount of the DNase enzyme is effective to prevent or ameliorate catabolic state leading to body weight loss associated with said chemotherapy.

In another variation, a method is disclosed for preventing or ameliorating bone marrow toxicity and/or catabolic changes in blood biochemistry associated with cytostatic and/or cytotoxic chemotherapy (e.g., doxorubicin therapy) in a subject suffering from a cancer and receiving or deemed to receive said chemotherapy, which method comprises administering to the subject a therapeutically effective amount of a DNase enzyme (e g., DNase I (e g., human recombinant DNase I or bovine pancreatic DNase I), analogues of DNase I (such as, e g., DNase X, DNase gamma, or DNAS1L2), DNase II, phosphodiesterase I, lactoferrin, or acetylcholinesterase), wherein said amount of the DNase enzyme is effective to prevent or ameliorate bone marrow toxicity and/or catabolic changes in blood biochemistry associated with said chemotherapy.

In yet another variation, a method is disclosed for preventing or ameliorating cardiotoxicity (e g., myocardial necrosis) associated with a cytostatic and/or cytotoxic chemotherapy (e g, doxorubicin therapy) in a subject suffering from a cancer and receiving or deemed to receive said chemotherapy, which method comprises administering to the subject a therapeutically effective amount of a DNase enzyme (e.g., DNase I (e.g., human recombinant DNase I or bovine pancreatic DNase I), analogues of DNase I (such as, e.g., DNase X, DNase gamma, or DNAS1L2), DNase II, phosphodiesterase I, lactoferrin, or acetylcholinesterase), wherein said amount of the DNase enzyme is effective to prevent or ameliorate cardiotoxicity associated with said chemotherapy.

In a further variation, a method is disclosed for preventing or ameliorating gastrointestinal toxicity associated with a cytostatic and/or cytotoxic chemotherapy (e.g., 5-fluorouracil/etoposide combination chemotherapy) in a subject suffering from a cancer and receiving or deemed to receive said chemotherapy, which method comprises administering to the subject a therapeutically effective amount of a DNase enzyme (e.g., DNase I (e.g., human recombinant DNase I or bovine pancreatic DNase I), analogues of DNase I (such as, e.g., DNase X, DNase gamma, or DNAS1L2), DNase II, phosphodiesterase I, lactoferrin, or acetylcholinesterase), wherein said amount of the DNase enzyme is effective to prevent or ameliorate gastrointestinal toxicity associated with said chemotherapy.

In another variation, a method is disclosed for preventing or ameliorating suppression of immunity associated with a cytostatic and/or cytotoxic chemotherapy (e.g., taxane therapy) in a subject suffering from a cancer and receiving or deemed to receive said chemotherapy, which method comprises administering to the subject a therapeutically effective amount of a DNase enzyme (e.g., DNase (e.g., human recombinant DNase I or bovine pancreatic DNase I), analogues of DNase I (such as, e.g., DNase X, DNase gamma, or DNAS1L2), DNase II, phosphodiesterase I, lactoferrin, or acetylcholinesterase), wherein said amount of the DNase enzyme is effective to prevent or ameliorate suppression of immunity associated with said chemotherapy.

In a further variation, a method is disclosed for preventing or ameliorating neutropenia associated with a cytostatic and/or cytotoxic chemotherapy (e.g., cyclophosphamide therapy) in a subject suffering from a cancer and receiving or deemed to receive said chemotherapy, which method comprises administering to the subject a therapeutically effective amount of a DNase enzyme (e.g., DNase I (e.g., human recombinant DNase I or bovine pancreatic DNase I), analogues of DNase I (such as, e.g., DNase X, DNase gamma, or DNAS1L2), DNase II, phosphodiesterase I, lactoferrin, or acetylcholinesterase), wherein said amount of the DNase enzyme is effective to prevent or ameliorate neutropenia associated with said chemotherapy.

In yet another variation, a method is disclosed for preventing or ameliorating a body weight loss associated with a radiation therapy in a subject suffering from a cancer and receiving said radiation therapy, which method comprises administering to the subject a therapeutically effective amount of a DNase enzyme (e.g., DNase I (e.g., human recombinant DNase I or bovine pancreatic DNase I), analogues of DNase I (such as, e.g., DNase X, DNase gamma, or DNAS1L2), DNase II, phosphodiesterase I, lactoferrin, or acetylcholinesterase), wherein said amount of the DNase enzyme is effective to prevent or ameliorate body weight loss associated with said radiation therapy.

In one variation of any of the above methods, the therapeutically effective amount of a DNase enzyme is at least 0.5 mg/kg/day or at least 1000 Kunitz units (KU)/kg/day, preferably at least 1.5 mg/kg/day or at least 3000 KU/kg/day. In one embodiment of any of the above methods, the therapeutically effective amount of a DNase enzyme is from 0.5 to 100 mg/kg/day or from 1000 to 200000 KU/kg/day, preferably from 0.5 to 50 mg/kg/day or from 1000 to 100000 KU/kg/day, more preferably from 1.5 to 50 mg/kg/day or from 3000 to 100000 KU/kg/day, most preferably from 10 to 50 mg/kg/day or from 20000 to 100000 KU/kg/day.

In one variation of any of the disclosed methods, the subject is human.

### Methods for Administering DNase and DNase Compositions

In the methods disclosed herein, a DNase enzyme can be administered before, during, and/or after the administration of a chemotherapeutic agent or during or after the administration of a radiation therapy. Preferably, (i) a DNase enzyme and a chemotherapeutic agent (or a radiation therapy) are administered at the same time, and/or (ii) a DNase enzyme is administered shortly after the administration of a chemotherapeutic agent (or a radiation therapy). DNase can be administered to the patient at one time or over a series of treatments; once or several times per day.

DNase doses useful in the methods disclosed herein depend on the type of chemotherapy or radiation therapy side effects to be treated, the severity and course of these side effects, previous therapy, the patient's clinical history and response to chemotherapy (or radiation therapy) and DNase, as well as the discretion of the attending physician. Non-limiting examples of useful dosage ranges include from 0.5 to 100 mg/kg/day or from 1000 to 200000 KU/kg/day, preferably from 0.5 to 50 mg/kg/day or from 1000 to 100000 Kunitz units (KU)/kg/day, more preferably from 1.5 to 50 mg/kg/day or from 3000 to 100000 KU/kg/day, most preferably from 10 to 50 mg/kg/day or from 20000 to 100000 KU/kg/day.

The administration of a DNase enzyme according to the methods disclosed herein can be performed by any suitable route, including systemic administration as well as administration directly to the site of the disease (e.g., to a primary tumor). Specific non-limiting examples of useful routes of administration include intravenous (IV), subcutaneous (SC), intraperitoneal (IP), oral, or by inhalation.

In certain embodiments, a DNase enzyme is formulated in a pharmaceutical composition with a pharmaceutically acceptable carrier or excipient.

The formulations used in the methods disclosed herein may conveniently be presented in unit dosage form and may be prepared by methods known in the art. The amount of active ingredients that can be combined with a carrier material to produce a single dosage form will vary depending upon the host being treated and the particular mode of administration. The amount of active ingredients that can be combined with a carrier material to produce a single dosage form will generally be that amount of the compound which produces a therapeutic effect.

In general, the formulations can be prepared with a liquid carrier, or a finely divided solid carrier, or both, and then, if necessary, shaping the product.

Pharmaceutical compositions suitable for parenteral administration may comprise one or more active ingredients (a DNase and, optionally, another compound effective for ameliorating or preventing side effects of a cytostatic and/or cytotoxic chemotherapy or a radiation therapy) in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents. Examples of suitable aqueous and nonaqueous carriers which may be employed in the pharmaceutical compositions of the disclosure include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions can also contain preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption, such as aluminum monostearate and gelatin.

Injectable depot forms can be made by forming microencapsule matrices of one or more active ingredients in biodegradable polymers such as polylactide-polyglycolide. Depending on the ratio of active ingredient to polymer, and the nature of the particular polymer employed, the rate of active ingredient's release can be controlled. Examples of other biodegradable polymers include poly(orthoesters) and poly(anhydrides). Depot injectable formulations are also prepared by entrapping the active ingredients in liposomes or microemulsions which are compatible with body tissue.

Formulations for oral administration can be in the form of capsules, cachets, pills, tablets, powders, granules, or as a solution or a suspension in an aqueous or non-aqueous liquid (e.g., as a mouthwash, as a composition to be swallowed, or as an enema), or as an oil-in-water or water-in-oil liquid emulsion, and the like, each containing a predetermined amount of one or more active ingredients.

In solid dosage forms for oral administration (capsules, tablets, pills, dragees, powders, granules, and the like), one or more active ingredients can be mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose, and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

Suspensions, in addition to one or more active ingredients, can contain suspending agents such as ethoxylated isostearyl alcohols, polyoxyethylene sorbitol, and sorbitan esters, microcrystalline cellulose, aluminum metahydroxide, bentonite, agar-agar and tragacanth, and mixtures thereof.

Powders and sprays can contain, in addition to one or more active ingredients, excipients such as lactose, talc, silicic acid, aluminum hydroxide, calcium silicates, and polyamide powder, or mixtures of these substances. Sprays can additionally contain customary propellants, such as chlorofluorohydrocarbons and volatile unsubstituted hydrocarbons, such as butane and propane.

### EXAMPLES

The present invention is also described and demonstrated by way of the following examples. However, the use of these and other examples anywhere in the specification is illustrative only and in no way limits the scope and meaning of the invention or of any exemplified term.

### EXAMPLE 1: Amelioration of acute doxorubicin toxicity in rats (reference example).

### Materials and Methods

42 Wistar male rats (180-200 g) were used in this experiment (obtained from Stolbovaya nursery of Russian Academy of Medical Sciences). Animals were kept under standard conditions with free access to food and drinking water. Animals were randomized into 7 groups (6 animals in each group) as follows:
1. Group I: control group (no Doxorubicin, no DNase);
2. Group II: animals treated by daily intravenous (IV) injections of Doxorubicin (LENS) at 3.75 mg/kg/day dose for 5 days plus daily intraperitoneal (IP) injections of human recombinant DNase I (Pharmsynthez OJSC) at 15 mg/kg/day dose (30000 KU/kg/day); Doxorubicin and DNase I injections were administered at the same time;
3. Group III: animals treated by daily IV injections of Doxorubicin (LENS) at 3.75 mg/kg/day dose for 5 days plus daily IP injections of placebo (water for injection [WFI]);
4. Group IV: animals treated by daily IV injections of Doxorubicin (LENS) at 7.5 mg/kg/day dose for 5 days plus daily IP injections of human recombinant DNase I (Pharmsynthez OJSC) at 15 mg/kg/day dose (30000 KU/kg/day); Doxorubicin and DNase I injections were administered at the same time;
5. Group V: animals treated by daily IV injections of Doxorubicin (LENS) at 7.5 mg/kg/day dose for 5 days plus daily IP injections of placebo (WFI);
6. Group VI: animals treated by daily IV injections of Doxorubicin (LENS) at 10.0 mg/kg/day dose for 5 days plus daily IP injections of human recombinant DNase I (Pharmsynthez OJSC) at 15 mg/kg/day dose (30000 KU/kg/day); Doxorubicin and DNase I injections were administered at the same time;
7. Group VII: animals treated by daily IV injections of Doxorubicin (LENS) at 10.0 mg/kg/day dose for 5 days plus daily IP injections of placebo (WFI).

Animals were monitored during 2 weeks from the beginning of experiment for survival and weight.

### Results

**Table 1: Animal survival data among different experimental groups**

| Group | I | II | III | IV | V | VI | VII |
|---|---|---|---|---|---|---|---|
| Number of dead/out of total in group | 0/6 | 1/6 | 3/6 | 3/6 | 5/6 | 6/6 | 6/6 |
| Median survival of deceased rats (in days) | - | 7 | 12±2.1 | 10.3±1.4 | 8.2 ± 1.0 | 6.2± 1.0 | 6.6 ± 0.9 |

The results summarized in Table 1 demonstrate that DNase suppresses the lethality of doxorubicin and increases the life span of rats challenged with sub-lethal doses of doxorubicin. The suppression of lethality, as measured by the number of surviving animals, occurred in a dose-dependent manner.

**Table 2: Body weight of surviving rats (in grams) following doxorubicin administration at 3.75mg/kg dose, measured before administration of doxorubicin and on Days 4, 7, and 14 of the treatment.**

| | Group I | Group II | Group III |
|---|---|---|---|
| Baseline | 167 ± 4 | 168 ± 4 | 164 ± 6 |
| Day 4 | 165 ± 5 | 160 ± 6 | 143 ± 6 |
| Day 7 | 172 ± 4 | 158 ± 5 | 151 ± 7 |
| Day 14 | 175 ± 4 | 164 ± 6 | 156 ± 6 |

The results summarized in Table 2 demonstrate that DNase treatment ameliorates body weight loss in rats who survived an acute doxorubicin challenge at the LD50 dose of 3.75 mg/kg.

**Table 3: Blood cell counts and blood biochemistry data from surviving rats following doxorubicin administration at 3.75mg/kg dose on Day 14 of treatment.**

| | Group I | Group II | Group III |
|---|---|---|---|
| Haemoglobin, G% | 13.5±0.5 | 11.9±0.4 | 9.5±0.7 |
| Hematocrit, % | 42.2±2.1 | 37.5±2.0 | 30.5±2.2 |
| RBC, 10iz/L | 7.8±0.2 | 6.8±0.2 | 5.7±0.4 |
| RBC sedimentation count, mm/h | 4.4±0.3 | 16.5±0.8 | 24.1±4.5 |
| Platelets, 10⁹/L | 540±25 | 440±25 | 340±25 |
| WBC 10^{9/L} | 7.7±0.4 | 6.0±0.3 | 4.9±0.2 |
| Segmented neutrophils, % | 29.6±1.3 | 13.5±1.0 | 11.6±1.3 |
| Basophils, % | 0 | 0 | 0 |
| Eosinophils, % | 0 | 0 | 2.4±0.3 |
| Monocyte, % | 2.7±0.5 | 2.9±0.4 | 2.3±0.5 |
| Lymphocytes, % | 63.4±1.7 | 80.3±1.4 | 82.3±2.2 |
| Plasmatic cells, % | 0.3±0.1 | 0.3±0.1 | 1.3±0.5 |
| Protein, g/L | 74.2±4.3 | 64.3±5.2 | 54.7±7.3 |
| Urea, mmol/L | 4.12±0.21 | 5.39±0.40 | 7.21±0.3 |
| Creatinine, Mmol/L | 54.7±6.2 | 65.5±7.2 | 68.3±8.4 |
| Glucose, mmol/L | 6.8±0.1 | 5.9±0.2 | 6.1±0.2 |
| Total lipids, g/L | 2.22±0.20 | 2.12±0.21 | 2.06±0.27 |
| Total cholesterol, mmol/L | 1.32±0.30 | 1.69±0.18 | 1.10±0.29 |
| Total bilirubin, mmol/L | 8.9±0.3 | 9.5±0.5 | 8.2±0.6 |
| Bilirubin bound, mmol/L | 2.0±0.4 | 2.6±0.3 | 2.0±0.5 |
| Na, mmol/L | 142±3 | 151±4 | 144±2 |
| K, mmol/L | 5.2±0.2 | 5.1±0.3 | 5.6±0.3 |

The results summarized in Table 3 indicate that DNase treatment ameliorates bone marrow toxicity and catabolic changes in blood biochemistry in rats who survived an acute doxorubicin challenge at the LD50 dose of 3.75 mg/kg.

The hearts deceased rats challenged with sub-lethal doses of doxorubicin (7.5 mg/kg) from (Groups IV and V) were autopsied. Serial myocardium microscopy samples from each heart were analyzed using an automated video analyzer to quantify the necrotic areas. The sum of necrotic area (Sₙₐ; nm²) was calculated as a sum of necrotic areas in 30 serial slides from each individual autopsied heart (n=3 for Group IV (Fig. 1, black bars); n=5 for Group V (Fig. 1, diagonal hatched bars)). Group IV received daily IP injections of human recombinant DNase I at 15 mg/kg dose, while Group V received daily IP injections of placebo (WFI).

The results presented in Figure 1 demonstrate that DNase treatment decreases the myocardial necrosis area in rats challenged with sub-lethal doses of doxorubicin.

### EXAMPLE 2: Amelioration of gastrointestinal toxicity of 5-f1uorouracil/etoposide combination chemotherapy (reference example).

### Materials and Methods

64 male Wistar rats (170-200 g) were used in this experiment (obtained from Stolbovaya nursery of Russian Academy of Medical Sciences). Animals were kept under standard conditions with free access to food and drinking water. On Day 1, Etoposide (LANCE) 200 mg/kg and 5-fluorouracil (5-FU; EBEWE) 400 mg/kg were given orally via feeding needle in 500 pl of a 9% glucose solution. Animals were divided into 4 groups of 16 rats each. Two hours after the Etoposide/5-FU challenge, rats were treated as follows:
1. Group I: IV placebo (WFI);
2. Group II: human recombinant DNase I (Pharmsynthez OJSC) at 1.5 mg/kg (3000 KU/kg) dose IV;
3. Group III: human recombinant DNase I (Pharmsynthez OJSC) at 50 mg/kg (100000 KU/kg) dose IV;
4. Group IV: cimetidine solution (Gedeon Richter) at 50 mg/kg dose IV.

36 hours later, all animals were euthanized and their stomachs were removed and analyzed for the presence of erosions and hemorrhages using an automated video analyzer system.

### Results

**Table 4: Effects of DNase I or cimetidine administration on gastrointestinal lesions in animals treated with 200 mg/kg Etoposide and 400 mg/kg 5-FU.**

| Gr. | Number of animals | Chemo | Treatment | Total number of lesions per group | Median number of lesions | Number of lesion free animals |
|---|---|---|---|---|---|---|
| 1 | 16 | Etoposide 200 mg/kg+ 5-FU 400 mg/kg per os | Placebo | 86 | 5.38±1.41 | 4 (25%) |
| II | 16 | Etoposide 200 mg/kg+ 5-FU 400 mg/kg per os | rHuDNase 1 1.5 mg/kg IV | 60 | 3.75±1.53 | 5 (31.25%) |
| III | 16 | Etoposide 200 mg/kg+ 5-FU 400 mg/kg per os | rHuDNase 1 50 mg/kg IV | 16 | 1.00±0.69 | 12 (75%) |
| IV | 16 | Etoposide 200 mg/kg+ 5-FU 400 mg/kg per os | Cimetidine 50 mg/kg IV | 40 | 2.5±1.1 | 10 (62.5%) |

The results summarized in Table 4 demonstrate that DNase suppresses chemotherapy-induced ulceration and increases the number of gastrointestinal erosion-free animals in a dose-dependent manner in animals treated with etoposide and 5-FU. DNase I dose of 50 mg/kg was significantly superior in terms of achieved protection, as compared to both the 1.5 mg/kg DNaseI dose and 50 mg/kg dose of cimetidine.

Figure 2 shows the stained stomach of a rat from Group III (A) and a rat from Group I (B). Multiple erosions and ulcers are visible in the stomach of the Group I animal, while the stomach from the Group III animal does not have any macroscopic abnormalities.

### EXAMPLE 3: Amelioration of taxane-induced suppression of immunity (reference examnle).

### Materials and Methods

10 male 6-week-old (CBAxC57Bl6) F1 mice (obtained from Rappolovo animal house) were given a single 20 mg/kg dose of paclitaxel (Paclitaxel-Teva) IV. A group of 5 paclitaxel treated mice were injected IP 1 hour later with recombinant mouse DNase gamma (USCN Life Science Inc.) at 2 mg/kg dose IP. A group of 5 untreated mice were injected IP at the same time with 1 ml of placebo (WFI) to serve as the control. All mice were killed 24 hours later by cervical dislocation. The thymuses from each group were collected and homogenized in a separator in RPMI 1640 media supplemented with 10% fetal calf serum. The suspension of thymocytes was filtered and sedimented in a centrifuge at 400xg for 2 minutes and then resuspended with the same media. Thymocytes were plated into 96 well plates (900,000 cells per well in 200 pl of RPMI 1640 supplemented with 10% fetal calf serum (FCS)) and incubated for 32 hours at 37°C, 100% humidity and 5% COz atmosphere. Each well was then supplemented with ³H thymidine (UC/10pl/well) and Concanavalin A (ConA; 50pl; 1.25 pg/ml). After 24 hours of further incubation, thymocytes were harvested and dried. Labeled ³H thymidine incorporation was measured using a Beckman scintillation
counter. The data on Con A-induced lymphocyte blast transformation is summarized in Table 5, below.

### Results

**Table 5: Effect of DNase gamma on Con A-induced lymphocyte blast transformation.**

| | Scintillation counts; CPM | |
|---|---|---|
| Group | Basal ³HT incorporation | Con A-induced ³HT incorporation |
| Control | 641±62 | 7894±1067 |
| Paclitaxel | 277±21 | 1686±142 |
| Paclitaxel + DNase | 357±25 | 5237±1232 |

The results summarized in Table 5 demonstrate that DNase ameliorates taxane-induced suppression of thymocytes' proliferative activity.

### EXAMPLE 4: Amelioration of cyclophosphamide-induced neutropenia (reference example).

### Materials and Methods

30 female 10-12 week old BALB/C mice (obtained from Rappolovo animal house) were used in this experiment. Animals were kept under standard conditions with free access to food and drinking water. On Day 1, neutropenia was induced by a single IP injection of 200 mg/kg cyclophosphamide (Cyclophosphanum-LANS) in 200 pl of WFI. Starting on Day 2, mice were then treated as follows:
1. Group I (n=10): control (no treatment);
2. Group II (n=10): animals were treated with human recombinant DNase I (Pharmsynthez OJSC) at 25 mg/kg/day (50000 KU/kg/day) dose injected IV on Days 2-5;
3. Group III (n=10): animals were treated with human recombinant GM-CSF (Neostim, Pharmsynthez OJSC) at 200 pg/kg/day dose injected subcutaneously (SC) on Days 2-5.

The white blood cell count (WBC) in each animal was monitored daily during the 10 day experiment using blood cell counter.

### Results

The graph in Figure 3 summarizes the dynamics of the white blood cell count (WBC) in the three experimental groups. The vertical axis represents WBC with units of 10⁹ WBC/L. The horizontal axis represents the number of days after cyclophosphamide injection.

The results in Figure 3 demonstrate that DNase ameliorates neutropenia induced by an alkylating agent such as cyclophosphamide. Of particular importance is that DNase treatment prevents the typical early phase decline in WBC count, while GM-CSF treatment is not able to prevent it.

### EXAMPLE 5: Synergy between DNase treatment and anticancer chemotherapy (reference example).

### Materials and Methods

To evaluate a possible synergy of combined use of DNase and nucleoside analog chemotherapeutic agent cytosine arabinoside (AraC), DBA2 mice (8-10 weeks old; 24-26 g; obtained from Rappolovo animal house) were transplanted IP with L1210 leukemia cells (1×10⁵ cells per mice; cells obtained from Petrov Institite of Oncology collection) on Day 0 to induce cancer. Treatment with DNase and AraC was initiated on Day 1 of experiment. Cytosine arabinoside (AraC; Cytosar; Pfizer) was injected IP at a dose of 1000 mg/kg on Days 2, 5 and 8. The injected doses were considered adequate for the model and injection route based on prior experiments (13-14). DNase II (Wornington) was injected IP at doses of 15 mg/kg/day and 45 mg/kg/day on Days 1, 3, 5, 8, 10, 12, 15, 17, and 19.

Animals were randomized into 6 experimental groups of 6 mice per group as follows:
1. Group I: negative control (no treatment);
2. Group II: positive control (animals treated with AraC);
3. Group III: animals treated with AraC and 15 mg/kg DNase;
4. Group IV: animals treated with AraC and 45 mg/kg DNase;
5. Group V: animals treated with 15 mg/kg DNase, no AraC;
6. Group VI: animals treated with 45 mg/kg DNase, no AraC.

### Results

Survival data are summarized in Figure 4. The vertical axis of Figure 4 represents the survival measured in days after implantation with the L1210 leukemia cells. The six experimental groups are represented on the horizontal axis: 1- Negative Control; 2 -AraC (positive control); 3 -AraC + DNase II 15 mg/kg; 4 -AraC + DNase II 45 mg/kg; 5 - DNase II 15 mg/kg; 6 - DNase II 45 mg/kg.

The results summarized in Figure 4 show that treatment with both AraC alone (Group II) and DNase II alone (Groups V and VI) results in better survival rates versus non-treated mice (Group I). However, the combination of AraC and DNase results in a clear survival advantage over either treatment alone. The synergistic effect of the combination treatment on survival rates was dose-dependent on increasing doses DNase.

### EXAMPLE 6: DNase treatment increases efficacy and decreases toxicity of cancer radiotherapy.

### Materials and Methods

To evaluate a possible synergy of combined use of DNase and radiation therapy, 60 SHR mice (12-14 weeks old; 28-32 g; obtained from Rappolovo animal house) were transplanted IP with Erlich carcinoma cells (1×10⁶ cells per mice; cells obtained from Petrov Institite of Oncology collection) on Day 0 to induce ascites tumor growth. Radiotherapy was performed using a cobalt-60 teletherapy machine. The abdominal fields of the mice were treated by external irradiation. The fractionation was 200 cGy per day for 5 days. Mice were divided into 6 groups treated as follows: Group 1: radiotherapy only (external irradiation 200 cGy per day for 5 days); Group 2: recombinant human DNAse 1 (Pharmsynthez) 20 mg/kg/day administered intramuscularly in 2 daily doses; Group 3: recombinant human DNAse I (Pharmsynthez) 40 mg/kg/day administered intramuscularly in 2 daily doses; Group 4: radiotherapy (external irradiation 200 cGy per day for 5 days) plus recombinant human DNAse I (Pharmsynthez) 20 mg/kg/day administered intramuscularly in 2 daily doses; Group 5: radiotherapy (external irradiation 200 cGy per day for 5 days) plus recombinant human DNAse I (Pharmsynthez) 40 mg/kg/day administered intramuscularly in 2 daily doses. Group 6 was a control group receiving placebo (water for injection).

### Results

Animal survival and dynamics of body weight on day 16 after transplantation of Erlich carcinoma are summarized in Table 6:

| Experimental Group | % Survival on Day 16 | Change in Median Body Weight on Day 16 |
|---|---|---|
| Group 1 | 40 | - 3g |
| Group 2 | 40 | +2 g |
| Group 3 | 50 | +1.5g |
| Group 4 | 80 | 0 |
| Group 5 | 100 | +1.5g |
| Group 6 | 0 | +5.5g |

The results summarized in Table 6 show that treatment with both radiation therapy alone (Group I) and DNase I alone (Groups 2 and 3) results in better survival rates versus non-treated mice (Group 6). However, the combination of radiation therapy and DNase results in a clear survival advantage over either treatment alone. The synergistic effect of the combination treatment on survival rates was dose-dependent on increasing doses of DNase. DNase treatment also clearly prevents the body weight loss induced by radiotherapy.

### References

- 1.: Joshi et al., 2007, J. Neurosci. Res. 85:497-503.
- 2.: Curigliano G. et al., Ann Oncol. 2012; 23 Suppl 7; vii155-vii 166.
- 3.: Syvak L.A. et al., Lik Sprava. 2012;(3-4):25-30.
- 4.: Verstappen C.C. et al., Drugs. 2003; 63(15): 1549-1563.
- 5.: Lyman G.H. et al., Oncology (Williston Park). 2006; 20(14 Suppl 9):16-25.
- 6.: Romiti A. et al., Cancer Chemother Pharmacol. 2013; 72(1):13-33.
- 7.: Laviano A. et al., N Engl J Med. 2012; 366:2319-2320.
- 8.: Gaurav K., Indian J of Med Paed Oncol., 2012; 33(1):13-20.
- 9.: Vincent D.T. et al., Cancer Chemother Pharmacol. 2013; 72(6):1157-68.
- 10.: Fushi W. et al., Cancer Res. 2013; 73:4256.
- 11.: Cools-Lartigue J. et al., J Clin Invest. 2013; 123(8): 3446-3458.
- 12.: Trejo-Becerril C. et al., PLoS One. 2012; 7(12): e52754.
- 13.: Chabot, G. and Momparier, R. L., Cancer. Treat. Rep. 1984; 68:1483-1487.
- 14.: Lech-Maranda, E. et al., Haematologica, 2000; 85:588-594.
- 15.: Hall IH, et al., J Pharm Sci. 1974; 63(4): 625-6.
- 16.: McBride A., et al, J Hematol Oncol. 2012; 5:75
- 17.: Basnakian et al., J. Am. Soc. Nephrol. 2005; 16: 697-702
- 18.: Gerber D., Am Fam Physician, 2008; 77(3):311-319

## Claims

1. A DNase enzyme for use in preventing or ameliorating at least one side effect of radiation therapy in a subject suffering from a cancer and receiving said radiation therapy, by preventing or ameliorating a toxicity associated with said radiation therapy, wherein the DNase enzyme is to be administered parenterally.

2. The DNase enzyme for use according to claim 1, wherein said side effect of said radiation therapy is selected from the group consisting of body weight loss, skin irritation or damage, fatigue, nausea, vomiting, fibrosis, bowel damage, memory loss, infertility, and a second cancer.

3. The DNase enzyme for use according to claim 1 or 2, wherein said radiation therapy is external beam radiation therapy or systemic radioisotope therapy.

4. The DNase enzyme for use according to any of claims 1 to 3, wherein the DNase enzyme is to be administered during a cycle of the radiation therapy.

5. The DNase enzyme for use according to any of claims 1 to 3, wherein the DNase enzyme is to be administered after a cycle of the radiation therapy.

6. The DNase enzyme for use according to any of claims 1 to 5, wherein the DNase enzyme is DNase I, preferably human recombinant DNase I.

7. The DNase enzyme for use according to any of claims 1 to 5, wherein the DNase enzyme is a DNase I analogue, preferably DNase gamma.

8. The DNase enzyme for use according to any of claims 1 to 5, wherein the DNase enzyme is a DNase II.

9. The DNase enzyme for use according to any of claims 1 to 8, wherein the DNase enzyme has an extended half-life.

10. The DNase enzyme for use according to any of claims 1 to 9, wherein the DNase enzyme is to be administered within the range of 0.5 - 50 mg/kg/day, preferably 1.5 - 50 mg/kg/day, still preferably 10 - 50 mg/kg/day.

11. The DNase enzyme for use according to any of claims 1 to 10, wherein the DNase enzyme is to be administered intravenously or subcutaneously.

12. The DNase enzyme for use according to any of claims 1 to 11, wherein the subject is human.

## Patentansprüche

1. DNase-Enzym zur Verwendung bei der Verhinderung oder Verbesserung mindestens einer Nebenwirkung der Strahlentherapie bei einem Subjekt, das an Krebs leidet und die Strahlentherapie erhält, durch Verhinderung oder Verbesserung einer mit der Strahlentherapie verbundenen Toxizität, wobei das DNase-Enzym parenteral verabreicht werden soll.

2. DNase-Enzym zur Verwendung nach Anspruch 1, wobei die Nebenwirkung der Strahlentherapie ausgewählt ist aus der Gruppe bestehend aus Körpergewichtsverlust, Hautreizung oder -schädigung, Müdigkeit, Übelkeit, Erbrechen, Fibrose, Darmschädigung, Gedächtnisverlust, Unfruchtbarkeit und einem zweiten Krebs.

3. DNase-Enzym zur Verwendung nach Anspruch 1 oder 2, wobei die Strahlentherapie eine externe Strahlentherapie oder eine systemische Radioisotopentherapie ist.

4. DNase-Enzym zur Verwendung nach einem der Ansprüche 1 bis 3, wobei das DNase-Enzym während eines Zyklus der Strahlentherapie verabreicht werden soll.

5. DNase-Enzym zur Verwendung nach einem der Ansprüche 1 bis 3, wobei das DNase-Enzym nach einem Zyklus der Strahlentherapie zu verabreichen ist.

6. DNase-Enzym zur Verwendung nach einem der Ansprüche 1 bis 5, wobei das DNase-Enzym DNase I ist, vorzugsweise humane rekombinante DNase I.

7. DNase-Enzym zur Verwendung nach einem der Ansprüche 1 bis 5, wobei das DNase-Enzym ein DNase-I-Analogon, vorzugsweise DNase gamma, ist.

8. DNase-Enzym zur Verwendung nach einem der Ansprüche 1 bis 5, wobei das DNase-Enzym eine DNase II ist.

9. DNase-Enzym zur Verwendung nach einem der Ansprüche 1 bis 8, wobei das DNase-Enzym eine verlängerte Halbwertszeit aufweist.

10. DNase-Enzym zur Verwendung nach einem der Ansprüche 1 bis 9, wobei das DNase-Enzym im Bereich von 0,5 bis 50 mg/kg/Tag, vorzugsweise 1,5 bis 50 mg/kg/Tag, noch bevorzugter 10 bis 50 mg/kg/Tag, verabreicht werden soll.

11. DNase-Enzym zur Verwendung nach einem der Ansprüche 1 bis 10, wobei das DNase-Enzym intravenös oder subkutan zu verabreichen ist.

12. DNase-Enzym zur Verwendung nach einem der Ansprüche 1 bis 11, wobei das Subjekt ein Mensch ist.

## Revendications

1. Enzyme DNase utilisée pour prévenir ou améliorer au moins un effet secondaire de la radiothérapie chez un sujet atteint d'un cancer et recevant ladite radiothérapie, en prévenant ou en améliorant une toxicité associée à ladite radiothérapie, l'enzyme DNase devant être administrée par voie parentérale.

2. Enzyme DNase à utiliser selon la revendication 1, dans laquelle l'effet secondaire de la radiothérapie est choisi dans le groupe constitué par la perte de poids, l'irritation ou les lésions cutanées, la fatigue, les nausées, les vomissements, la fibrose, les lésions intestinales, la perte de mémoire, l'infertilité et un second cancer.

3. Enzyme DNase à utiliser selon la revendication 1 ou 2, dans laquelle ladite radiothérapie est une radiothérapie par faisceau externe ou une thérapie systémique par radio-isotopes.

4. Enzyme DNase utilisée selon l'une des revendications 1 à 3, dans laquelle l'enzyme DNase doit être administrée pendant un cycle de la radiothérapie.

5. Enzyme DNase utilisée selon l'une des revendications 1 à 3, dans laquelle l'enzyme DNase doit être administrée après un cycle de radiothérapie.

6. Enzyme DNase utilisée selon l'une des revendications 1 à 5, dans laquelle l'enzyme DNase est la DNase I, de préférence la DNase I recombinante humaine.

7. Enzyme DNase à utiliser selon l'une des revendications 1 à 5, dans laquelle l'enzyme DNase est un analogue de la DNase I, de préférence la DNase gamma.

8. Enzyme DNase à utiliser selon l'une des revendications 1 à 5, dans laquelle l'enzyme DNase est une DNase II.

9. Enzyme DNase à utiliser selon l'une des revendications 1 à 8, dans laquelle l'enzyme DNase a une demi-vie prolongée.

10. Enzyme DNase utilisée selon l'une des revendications 1 à 9, dans laquelle l'enzyme DNase doit être administrée à raison de 0,5 à 50 mg/kg/jour, de préférence 1,5 à 50 mg/kg/jour, de préférence encore 10 à 50 mg/kg/jour.

11. Enzyme DNase à utiliser selon l'une des revendications 1 à 10, dans laquelle l'enzyme DNase doit être administrée par voie intraveineuse ou sous-cutanée.

12. Enzyme DNase utilisée selon l'une des revendications 1 à 11, dans laquelle le sujet est humain.
